# EUROPEAN PATENT APPLICATION

(11) **EP 4 205 559 A1**
(43) Date of publication of application: **05.07.2023**
(21) Application number: 22210905.0
(22) Date of filing: 01.12.2022
(51) Int. Cl.: A23L 33/105, A23L 19/00, G09B 19/00, A23L 33/00

(54) **PERSONALISED FUNCTIONAL NUTRITIONAL PRODUCT**

(30) Priority: 29.12.2021 NL 2030332
(71) Applicant: MiFood B.V., 5928 SZ Venlo (NL)
(72) Inventor: NOLET, Raymond Philippe Hubert Etienne, 5928 SZ Venlo (NL)
(74) Representative: V.O.

(57) **Abstract**

The invention relates to a method for determining a recommended functional nutritional product for an individual person based on health data of that person, wherein
a) one or more health data of the person are provided, said health data comprising a content of at least one phytochemical, preferably a plurality of phytochemicals, in a blood sample of the person;
b) said health data are compared with recommended health data;
c) one or more plant-based nutritional product ingredients are selected for the recommended food product on the basis of the recommended health data, said ingredients containing phytochemicals; and
d) the functional nutritional product is formulated from said one or more plant-based nutritional product ingredients, which recommended functional nutritional product is plant-based.

## Description

The invention relates to the field of functional food products. In particular, the invention provides a method for determining a recommended nutritional product for an individual user based on individual health data. The invention further relates to a plant-based nutritional product, for use in increasing resistance against developing a medical disorder, reducing the risk of developing a medical disorder in a human or for alleviating a symptom of a medical disorder in a human diagnosed with said medical disorder. The invention further relates to a process for preparing a plant-based nutritional product. The invention further relates to a plant-based nutritional product.

Functional food products are usually designed to have physiological benefits and/or reduce the risk of chronic disease beyond basic nutritional functions. When formulating a functional food product it is common to add one or more potentially beneficial bioactive compounds to an otherwise conventional food. Such bioactive compounds used in functional food products are typically isolated from a natural source or synthetic.

Fruits and vegetables have since long been recognized as suitable sources for various bioactive components, such as - for example - vitamins, fibres, antioxidants, proteins, fatty acids and minerals. In particular, evidence exists that the consumption of fruits and vegetables can (help to) protect against the development of diverse chronic diseases, such as cardiovascular diseases, diabetes type 2 and cancer (WCRF Second Expert Report 2007).

E.g., Thompson et al. reported that a diet including foods from 18 botanical families induced a significant reduction in DNA oxidation and concluded that smaller amounts of many phytochemicals may have greater beneficial effects than larger amounts of fewer phytochemicals.

Wilms et al (Carcinogenesis, vol 28 no 8 pp1800-1806, 2007) reported the protective effects of blueberry/apple- juice against de inductions of genetic damage and its potential to induce molecular mechanisms reflecting a reduction of the risk of developing diseases.

Erlund et al. (Am J Clin Nutr 2008; 87 323-331) reported favourable effects of berry consumption on platelet function, blood pressure and HDL cholesterol.

In a review by Van Breda and De Kok (Mol. Nutr. Food Res. 2018 Jan;62(1)) an update of the scientific literature on additive and synergistic effects of mixtures of phytochemicals is provided. Most research has been carried out in in vitro systems in which synergistic or additive effects have been established on the level of cell proliferation, apoptosis, antioxidant capacity, and tumour incidence, accompanied by changes in gene and protein expression in relevant pathways underlying molecular mechanisms of disease prevention. The number of human dietary intervention studies investigating complex mixtures of phytochemicals is relatively small. These studies support that combining transcriptomic data with phenotypic markers provide insight into the relevant cellular processes which contribute to the antioxidant response of complex mixtures of phytochemicals.

However, processing of the raw plant product (for obtaining a fraction, e.g. juice thereof, for isolating one or more bioactive components of interest or for use as a processed whole ingredient) can have a significant effect on the contents of the bioactive components. For instance, various bioactive compounds are thermally instable, whereby a relatively limited exposure to heat can already result in significant destructions. Other bioactive components require extreme temperatures to become bioavailable. Oxidation and exposure to light are also important causes of loss of bioactive components.

Regular commercial food production is generally mass production. The inventors realised that besides cost factors, the production capacity, the production speed and the need for mass-availability of the ingredients can contribute to a relatively low content of desirable bioactive components in the final product. At a high production capacity and/or speed less care may be taken to avoid hot spots in the production process or exposure to oxygen and light, which can each be causes of break-down of bioactive ingredients. High temperatures are regularly used to make the end product germ-free and thus safe for consumption. This compensates for unhygienic working in production.

The inventors further realised that the bioactive component compositions of different batches of the same species or even variety of fruits or vegetables can vary considerably, whereby also in the absence of significant breakdown of bioactive components prior to consumption, the actual consumption of desirable amounts of important bioactive components is not controlled or even guaranteed, even when daily quantities of fruits and vegetables are consumed following the guidelines of authorities, such as the World Health Organisation or Voedingscentrum, the Netherlands (e.g. via voedingscentrum.nl). This problem also occurs in methods providing a personalised dietary plan, recommending recipes but not actually delivering a physical food product to the person in need thereof.

E.g., in US2013/157233A1 it is stated that various phytochemicals can be beneficial in the treatment of a healthcare condition. A method is proposed for preparing a health-condition specific personal eating plan based on personal healthcare information, such as such as dietary information, current medications, pre-existing health conditions biometrics and eating behaviours. Further, the method comprises generating a list of foods, which appear to be regular commercially available foods, to incorporate in the eating plan based on the health condition and the phytochemical content and density **of the foods.** It is not disclosed to use a content of phytochemicals in a blood sample, retinal blood vessel condition data, a gene expression profile, sensitivity to DNA damage of the person as healthcare information. Further, the plan typically is an instruction for meals, containing several separate components, which meal does not need to be plant-based but can contain dairy products, meat or fish, rather than a formulated functional nutritional product. Further, a process for preparing a mashed plant-based functional nutritional food product involving pascalisation or vacuum coating is not disclosed either. Rather, recommended meals are prepared in a conventional way to prepare meals, including cooking (e.g. roasting, boiling, poaching) courses. The publication does not contain any experimental support.

The inventors further realised that conventional industrial production of food products, including functional food products, does generally not take into consideration the consumers at an individual level. Different individuals may react differently to changes in food and the extent to which different individuals' health benefits from a specific food product depends on specific genetic characteristics. Moreover, the inventors came to the insight that besides genetic factors also health status of an individual (e.g. sufficient beneficial bacteria in the small intestines) and the actual content of various phytochemicals in the individual's blood form a relevant factor for determining which composition of a food product is recommendable.

The inventors thus concluded that it would be desirable to recommend food products comprising phytochemicals to consumers on an individual level on the basis of certain health data from the individual to which the food product is recommended.

The inventors further found that hereby it is possible to provide a health benefit, such as increasing resistance against developing a medical disorder in a human, reducing the risk of developing a medical disorder in a human or for alleviating a symptom of a medical disorder in a human diagnosed with said medical disorder.

Accordingly, the present invention relates to a method for determining a recommended nutritional product for a person based on individual health data, wherein
a) health data of the person are provided, said health data comprising a content of one or more phytochemicals in a blood sample of the person;
b) said health data are compared with a recommended health data profile, thereby obtaining a 'deviation-from-target' profile;
c) one or more, preferably two or more, plant-based nutritional product ingredients comprising phytochemicals are selected on the basis of the 'deviation from target' profile and the recommended health data profile; and
d) the recommended nutritional product is formulated from said plant-based nutritional product ingredient or ingredients, which recommended nutritional product is plant-based.

The health data are typically obtained by measuring the health data. This can generally be done in a manner known per se.

The recommended nutritional food product is typically formulated to help to adjust the blood concentration of one or more phytochemicals, preferably a plurality of phytochemical concentrations in blood, into a recommended range or to maintain the blood concentration of one or more phytochemicals, preferably a plurality of phytochemical concentrations in blood within a recommended range. In principle there can already be a benefit in determining the concentration of a single phytochemical and using those data for recommendation, in particular in combination with one or more health data types, e.g. gene expression profile data or microbiome data. A plurality of phytochemicals can be at least two, at least three, at least four, at least five, at least six, at least seven, at least eight, at least nine or at least ten. There is in principle no maximum; if desired the content of 500 other more phytochemicals may be determined. In practice however, good results are already achieved with content data of less than 100 phytochemicals in blood, less than 50 phytochemicals in blood, 25 or less phytochemicals in blood or 10 or less phytochemicals in blood, in particular 1, 2, 3, 4, 5 or 6 phytochemicals. A surprisingly good recommendation is already possible based on a single class of phytochemicals, such as carotenoids, as exemplified in Example 1. Herein a recommendation is based on the level of six carotenoids, which may be grouped into four groups, in blood.

Typically, further health data that are advantageously provided in a method according to the invention comprise at least one of the following, preferably two, three, four or all five of the following
- a gene-expression profile of the user;
- retinal blood vessel condition data of the user;
- data regarding the sensitivity to DNA damage upon exposure to oxidative stress of blood cells of the user;
- microbiome data of the user;
- further health data regarding the functioning of the gastro-intestinal tract.

In an embodiment, further health data comprise bodyweight or body mass index. Besides health data other factors can be taken into consideration, if desired, e.g. age or gender. In a further embodiment, physical activity level is taken into consideration.

The invention further relates to a plant-based nutritional product, for use in increasing resistance against developing a medical disorder in a human, reducing the risk of developing a medical disorder in a human or for alleviating a symptom of a medical disorder in a human diagnosed with said medical disorder, wherein the plant-based nutritional product is formulated using a method according to the invention and the formulated nutritional product is to be administered to said human. In particular, the medical disorder may be a disease of affluence.

The invention further relates to a method of treating a human, comprising administering an effective amount of the plant-based nutritional product administered is formulated using a method according to the invention, thereby increasing resistance against developing a medical disorder in a human, reducing the risk of developing a medical disorder in a human or for alleviating a symptom of a medical disorder in a human diagnosed with said medical disorder.

The invention further relates to the use of the recommended plant-based nutritional product as nutrition or as a nutritional supplement for a human, wherein the plant-based nutritional product to be administered is formulated using a method according to the invention.

The invention further relates to a process for preparing a plant-based nutritional product, which plant-based nutritional product is as defined elsewhere herein for a method according to the invention, which plant-based nutritional product preferably is a recommended functional nutritional product determined using a method according to the invention, wherein the process comprises
- selecting one or more, preferably two or more nutritional product ingredients selected from the group consisting of vegetables, fruit parts and vegetable parts;
- mashing the selected product ingredients;
- when having selected two or more product ingredients: blending the selected product ingredients before, during or after mashing;
- subjecting the mashed product ingredient(s) or the mashed blend to pascalisation thereby obtaining a pascalised mash, preferably a pascalised blend;
- optionally further processing the pascalised mash, preferably the pascalised blend e.g. on a dry carrier;
- thereby obtaining the plant-based nutritional product;
which process is carried out at a temperature of about 35 °C or less, preferably a temperature in the range of 1-30 ⁰C.

Usually, the mash, preferably the blend, is packaged and then pascalised (e.g. about 6000 bar during 3 minutes at a low temperature, e.g. of about 22 °C or less ) in its packaging. After packaging the product is typically ready to be consumed. For a dry product, drying is typically done after pascalisation. Advantageously, the pascalised mash is immobilised on a dry carrier material, dried and then packaged. Applying and drying of the mash is advantageously done using vacuum coating, which allows penetration of the mash into a porous carrier material and a high degree of loading the carrier material with the mash.

A nutritional product as obtained in accordance with the invention is in particular advantageous in that the bioactivity of phytochemicals present in the ingredients is at least substantially preserved during its production. Loss of phytochemicals may vary to a certain extend dependent on their sensitivity to temperature changes, oxidation or other side reactions. Still, loss of phytochemicals in a product produced in accordance with the invention is limited, with generally a loss of only 25 % or less, after three weeks of storage in a refrigerator (at about 4 ⁰C) and - on average - a loss of 50 % or less, 12 weeks after storage in a refrigerator (at about 4 °C). It was also found that without pascalisation, the phytochemical content was less well preserved during storage.

In accordance with the invention, it is further advantageous that the plant-based nutritional product can be used as a supplement to the regular meals consumed by a person. In contrast to a method wherein a person has to follow a dietary plan, requiring acquiring and/or preparing various meals (or meal ingredients), the present invention offers a plant-based nutritional product that can be directly consumed as a single product, whilst providing recommended phytochemicals. In accordance with the invention, the person does not need to pay special attention to (changing) the regular meals the person is used to take in order to comply with the dietary plan. The nutritional products in accordance with the invention can thus easily as a supplement to a person's habitual meals. This facilitates consumption compliance. Although the plant-based nutritional product can advantageously be consumed separate from regular meals (breakfast, lunch, dinner), it can also be used as a meal (e.g. breakfast) or part thereof (e.g. dessert after a main course).

### Brief description of the Figures:

Figure 1 schematically shows an experimental set up for testing an effect of consuming a recommended nutritional product in accordance with the invention.
Figures 2 and 3 show effects of supplementing humans' diets with a nutritional product in accordance with the invention on retinal health data.
Figure 4 shows the 35 generally most abundant genera in human microbiome.

The nutritional product determined, recommended, formulated or prepared in accordance with the invention is plant-based, i.e. it is generally suitable for consumption by vegetarians and vegans. Thus, it is generally essentially free of ingredients from animal origin. Thus, it is generally essentially free of synthetic additives. The nutritional product can typically be considered a functional food product, i.e. a food product containing an effective amount of phytochemicals to support a healthy diet, in particular a functional whole food product.

The intended user for which the recommended food product is determined and - if desired - prepared, is generally a human, in particular an adult human.

It is important to determine (measure) a phytochemical content in blood, because this is a measure for the amount of the phytochemical that actually has been absorbed (digested) by the body. If not absorbed into the bloodstream, the effect of phytochemicals in the product is generally limited to some form of action in the gastro-intestinal tract, e.g. as a nutrient to the microbiome. A lower than expected content of a phytochemical in the blood can also be a sign of an unsatisfactory functioning of the gastro-intestinal tract, e.g. due to an unsatisfactory microbiome composition. Microbiome may disbalanced in that levels of certain bacteria or a too high or too lows. Within the context of the present invention, the microbiome can also be analysed and health data resulting from such analyse can also serve as a factor to be taken into consideration when formulating a nutritional product in accordance with the invention. On the basis of this, one or more specific vegetables or fruits can be selected as an ingredient for the recommended food product. For instance chicory can be recommended as an ingredient in case of a disbalanced microbiome. It is thought that inter alia the presence of inulin in chicory is advantageous in case of a disbalanced microbiome.

The contents of at least one phytochemical, preferably a plurality of phytochemicals, in a blood sample of the person can be determined in a manner generally known per se for the phytochemicals of interest, e.g. using HPLC (high pressure liquid chromatography), GC (gas chromatography), electrophoresis and/or mass spectrometry (MS) or a combination of these techniques. The blood sample can be a venous blood sample. This can be provided in a conventional way. In practice, A blood sample of the person for determining a phytochemical content is conveniently provided by a remote microsampling device. This generally provides sufficient blood for determining phytochemical contents and can be provided by the person for whom the nutritional product is recommended without interference of a health care professional. It also allows sending the sample by mail or the like from the person to a test lab where the sample is to be analysed. Particularly suitable is a volumetric absorptive microsampling device, such as a Mitra^{®} sampling device (available from Neoteryx).

Useful phytochemicals of which the content in the blood sample is determined and/or which are advantageously used as a selection criterion for the nutritional product ingredients to be used for formulating and/or preparing the functional nutritional product are usually selected from the group consisting of (poly)phenolic compounds (in particular flavonoids, phenolic acids and lignans), isoflavonoids, anthocyanins (a.k.a. anthocyans), diallyl sulphide, phytoestrogens (in particular estrogenic isoflavones), flavanones, flavanoles, terpenoids, carotenoids (in particular lycopenes; carotenes (alpha-carotene, beta-carotene); lutein and zeaxanthin as a members of the xanthophyll group, beta-cryptoxanthin, zeaxanthine), limonoids, isoflavonoids, phytosterols, glucosinolates, mono-unsaturated fatty acids, polyunsaturated fatty acids, caffeine, quaternary ammonia compounds (in particular carnitine and/or choline), co-enzyme Q10, creatine, dithiolthiones, prebiotics, taurin and vitamins (such as vitamin A, vitamin D, vitamin E).

In general, preferred phytochemicals of which at least the content is determined are one or more phytochemicals selected from the group of carotenoids, polyphenols, anthocyanines (a.k.a. anthoyanes), phytosterols, phyto-estrogens, Co-enzyme Q10 and glycosinulates. It is particularly preferred to determine the contents of carotenoids, more in particular of carotenoids, polyphenols, anthocyanines ánd glycosinulates.

Anthocyanins are responsible at least in part for the blue, purple or red colour of many plant tissues. They are powerful antioxidants and have antiinflammatory activity. The presence of anthocyanins in a nutritional product (for use) according to the invention is in particular preferred for improving resistance against a cardiovascular disease. Further, they add to a protective effect for retinal microvascularisation. Raspberries, blueberries, apples, cherries, grapes and, strawberries are particular suitable sources for anthocyanins, but other fruits or vegetables may also contain anthocyanins, e.g. onions.

Co-enzyme Q10 plays an important role in the energy production of cells. Further it is an antioxidant. It is generally produced by the human body. However, in accordance with the invention an ingredient may be selected for formulating the nutritional product, which ingredient provides a source of Co-enzyme Q10, such as spinach. In the human body, two forms exist: ubiquinone (oxidised form) and ubiquinol (reduced form), the latter having antioxidant activity. Supplementation with Co-enzyme Q10 can be recommended in particular if the ratio ubiquinol to ubiquinone is relatively low. Further, an increased demand of Co-enzyme Q10 may arise in humans that are highly physically active (such as people that exercise intensely, people that generally perform heavy labour) and in aging humans. Medical indications for supplementation with Co-enzyme Q10 are dyslipidaemia, hypertension, endothelium disfunction, atherosclerosis, heart failure, cardiomyopathy, myocardial infarct, metabolic syndrome, diabetes, chronic inflammation, multiple sclerosis, steatosis (not related to alcoholism), neurodegenerative disorders, reduced fertility, autistic spectrum disorders, fibromyalgia, chronic fatigue syndrome, migraine and kidney disorders.

Carotenoids (precursor of vitamin A) are powerful antioxidants and can have various physiological benefits and be advantageous in relation to various medical disorders. E.g. beta-carotenes and lutein are beneficial for eye functioning; for instance they can contribute to a delay in the onset of macular degeneration. Lycopenes may be used in the prophylaxis or in the treatment of a person suffering from a cardiovascular disorder. Further, carotenoids may contribute to increasing resistance against developing cancer or metabolic syndrome, reducing the risk of developing a cancer or metabolic syndrome or for alleviating a symptom of cancer or metabolic syndrome. Furthermore, considering the strong indications in the art that a diet rich in fruit and vegetables is protective against various diseases, including cardiovascular diseases, metabolic syndrome, insulin resistance, diabetes mellitus type 2 and cancers, the inventors realised that carotenoid level in blood, in particular the levels of lycopenes, alpha-carotene, beta-carotene, lutein, beta-cryptoxanthin and zeaxanthin form a good marker for dietary fruit and vegetable intake of a person. Accordingly, usually the method for determining a recommended nutritional product according to the invention comprises providing the total of the carotenoids in the blood of a person and/or the individual levels of lycopenes, alpha-carotene, beta-carotene, lutein, beta-cryptoxanthin and zeaxanthin and comparing these health data with recommended levels (recommended health data). On the basis of these results a selection of fruit/vegetable ingredients can be made dependent on carotenoid levels. In practice, it is not necessary to determine and recommend on an individual level for each of the carotenoids. Particularly good results have been achieved with providing the blood levels and determining recommended levels for up to four marker groups: 1. the carotenes, typically alpha-carotene and beta-carotene (in particular a marker for yellow and orange vegetables); 2. lycopenes (in particular a marker for dietary red vegetable, e.g. tomato, consumption); 3. beta-cryptoxanthin (in particular a marker for dietary fruit consumption); 4. the total level of lutein and zeaxanthin taken together is in particular a marker for dietary green vegetable consumption . In general, a recommended total carotenoid level in blood is at least 1.5 µM, because a content below 1.5 µM is considered a high risk factor for adverse health effects; it is considered a strong indicator for a much too low fruit and vegetable consumption by the person, an inadequate digestion of consumed fruits and vegetables or a serious lack of variety in the person's fruit and vegetable intake high health risk. In particular it is recommended that the total carotenoid content in blood is at least 2.5 µM, for a low risk of an adverse health effect. More preferably the recommended total carotenoid level in blood is at least about 4 µM, for a particularly low risk of an adverse health effect, or in terms of activity: for a high effectivity in improving the resistance against a medical disorder, reducing the risk of developing a medical disorder, or treating a symptom of a medical disorder. An upper limit is not particularly critical. At very high levels yellow or orange discoloration of the skin (carotenemia) may occur, which in itself is a benign condition, but may be undesired from a cosmetic perspective. In practice, the maximum recommended level of total carotenoids in blood is thus usually at a level not leading to carotenemia. The recommended level is generally less than 20 µM, in particular about 10 µM or less, about 8 µM or less or about 6 µM or less. If recommendations are made for the four groups of carotenoids, the recommendation is generally that the levels of carotenes and lycopenes are each at least about 0.5 µM, in particular at least about 0.7 µM , more in particular at least about 0.9 µM and that the total and the total of luteïne plus zeaxanthin both are at a concentration of at least about 0.2 µM in particular of at least about 0.4 µM, more in particular at least about 0.5 µM, with the proviso that the total is in recommended range too. In Example 1 an example is given of a specific case. It is in particular preferred to additionally determine the vitamin A content, the vitamin D content and the vitamin E content in the blood. Further, it is in particular preferred to additionally determine, co-enzyme Q10 (ubiquinone-10) and/or the ratio ubiquinol to ubiquinone-10 (ratio Q10).

Phytochemicals having antioxidant activity are generally useful to help to reduce the risk of developing cancer, for instance because their protective effect on DNA damage. However, they may also have a positive effect to help to reduce the risk of developing a cardiovascular disease or reduce the risk of or severity of oxidative damage to various tissues. Phyto-sterols are in particular useful for a use related to cardiovascular diseases. Phyto-oestrogens are in particular useful to avoid or reduce osteoporosis, especially - but not only - for women in or after menopause.

The contents of selected phytochemicals can be compared with a normal (healthy) concentration range in blood. If the measured content of a phytochemical is too high, a food can be recommended which has a low content of said phytochemical or is essentially free thereof. If the measured content of a phytochemical is too low, a food can be recommended which has a high content of said phytochemical. Unless specified otherwise herein, a normal concentration range can be based on common general knowledge, e.g. a clinical chemistry handbook or a publication cited herein.

It is particularly preferred to use a gene expression profile of the person for which the nutritional product is recommended. The gene expression profile can give additional insight in which kinds of phytochemicals are likely to have a particularly positive health effect. The gene expression profile can be provided in a manner known per se, for instance making use of a single cell gel electrophoresis assay (such as CometAssay^{®}). mRNA from one or more white blood cells is typically used as material to obtain a gene expression profile from.

Data regarding the sensitivity to DNA damage upon exposure to oxidative stress of blood cells of the user DNA damage data of the user can be obtained by provoking white blood cells isolated from a blood sample of the user with hydrogen peroxide and determining the sensitivity for cleavage of bonds in the DNA strand. A high sensitivity to DNA damage is an indicator for determining/formulating a food product with a relatively high content.

Retinal blood vessel condition data of the user are in particular useful to get better insight in the risk of developing a cardiovascular disease or diabetes or a better insight in the progress thereof in a person suffering from such a disease. Even though general focus is conventionally on monitoring status of primary large vessels like the conduit arteries (macro circulation), the microcirculation involvement is crucial in many cardiovascular conditions. In addition of being an early marker for cardiovascular disease, microvascular changes could also be a primary cause for the development of cardiovascular changes. More specifically, narrower retinal arteriolar calibre and wider venular calibre are markers of poorer microvascular health and could predict systemic vascular diseases, including hypertension, diabetes, stroke and cardiovascular disease. Arterial narrowing is seen in hypertension, CAD in women (and associated with diabetes risk, larger venules are seen in obesity, hypertension or diabetic retinopathy. The microvasculature of the retina can be easily viewed and non-invasively imaged, which makes it an attractive surrogate marker for measuring effects on the systemic microvasculature.

Microbiome data can be obtained in a conventional manner (such as cell counts of known beneficial and potentially pathogenic micro-organisms). Microbiome data provide a useful insight in the functioning of the gastro-intestinal tract and a poor ratio between beneficial micro-organisms (such as various *bifidobacteria* and *lactobacilli*) and potentially pathogenic micro-organisms (e.g. *E. coli)* be used in particular in relation to gastro-intestinal disorders. However, growing evidence exists that the microbiome is relevant to a vast array of other disorders. Further, a healthy gut flora is beneficial to digestive processes and is in particular thought to contribute to an improved uptake of phytochemicals from the intestines into the blood stream, in particular when the administered food product comprises a substantial amount of whole vegetable/fruit ingredients. In such ingredients, a degradation of the food matrix, such as cells or fibrous structures is usually important to make phytochemicals available for uptake into the bloodstream.

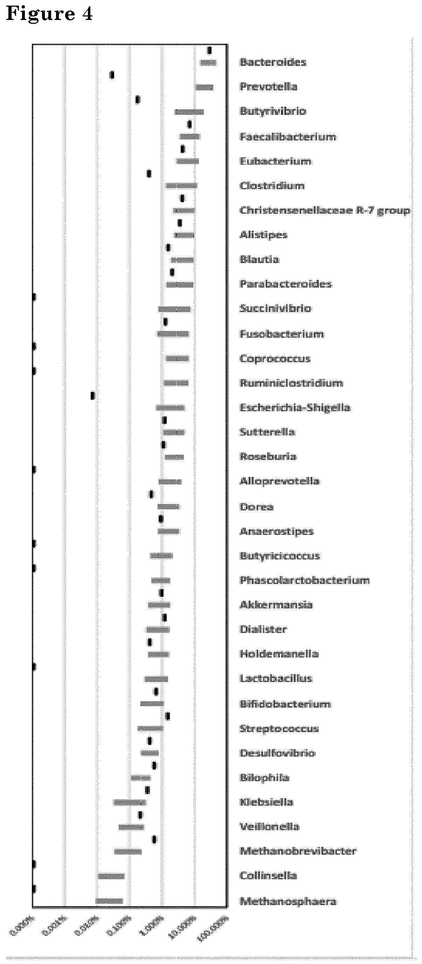

In a method of the invention, microbiome data may be provided before a first consumption of a food product recommended in accordance with the invention. In practice this is not necessary though. Microbiome data are preferably provided after a user has started to consume the recommended food product. For instance if the beneficial effects of determining a recommended products on the basis of blood data (phytochemical content, genetic expression, sensitivity to DNA damage) are relatively small, insight in quality (insight in which species of microorganisms are present) and quantity (numbers of the different microorganisms) in the microbiome can help to identify a reason. A general indicator for a good quality of the microbiome is a high diversity of different bacterial genera; over 500 bacterial genera have been determined in human intestinal flora. These include beneficial bacteria, such as various lactic acid producing bacteria (*lactobacilli, bifidobacteria*) but also potentially pathogenic bacteria (e.g. *Escherichia*)*.* A high diversity is generally already a useful indicator for a stable microbiome that is functioning satisfactorily. The diversity can be considered high when there are at least 265 different bacterial genera in a faecal sample. If desired, one may analyse in more detail the relative presence of the generally most abundant bacterial genera, in particular of 5-35, 10-35 or 25-35 abundant bacterial genera. Figure 4 shows the 35 generally most abundant genera; the horizontal bars show a typical range for their general abundance in highly diverse intestinal flora. The dots show the abundance in a specific faecal specimen of a human, whose microbiome actually showed high diversity (343 genera out of 550 that were screened for). Furthermore, one may screen for specific bacteria that have been associated with a risk of developing colonic cancer or for specific bacteria that have been associated with risk of infection. Also one may evaluate the abundance of beneficial lactic acid producing bacteria, in particular *bifidobacteria* and *lactobacilli.* Moreover, determining the abundance of methane producing bacteria, such as *methanobrevibacter* and *methanosphaera* provides useful health data. On the one hand, such bacteria can have a net effect of reducing gas volume because they can convert carbon dioxide and hydrogen into methane, reducing the risk of intestinal expansion by the gas. On the other hand, they may contribute to a reduction in peristaltic movement, which may be a cause of constipation. A group of undesired bacteria are those associated with a high fat consumption.

E.g. with a malfunctioning microbiome, digestion of the food product can be poor and thereby passage of phytochemicals provided by the recommended food product from the intestines into the blood stream can be reduced. This can then be addressed by reformulating a recommended food product, e.g. by adjusting the fibre content (qualitatively and/or quantitatively) and/or recommending probiotics. If probiotics are advisable to be included in a person's diet, as may be the case due to a low abundance of lactic acid producing bacteria, probiotics are typically recommended to be administered separate from the plant-based nutritional product. In particular, the plant-based nutritional product is generally produced and packaged without including probiotics. A high abundance of bacteria associated with cancer or infection may be used to select a food product ingredient rich in one or more phytochemicals contributing to the protection against the development of cancer respectively infection. Besides ingredients rich in antioxidants, fibrous ingredients and ingredients rich in seeds are in particular considered beneficial in this respect.

In an embodiment, further health data regarding the functioning of the gastro-intestinal tract are provided. Like the microbiome data, these can be provided for a first determination of a recommended product or they can be provided after consumption of a recommended product has already started, e.g. if beneficial effects appear to be relatively small. Advantageously use is made of an in vitro model of (a part of) the gastro-intestinal tract. Such models are known in the art. Particular suitable is a TIM System (available from TNO, the Netherlands). TIM-1 is a validated, dynamic, computer-controlled in vitro model of the stomach and small intestine that is suitable to study bio-accessibility of digestion products or phytochemicals. It accurately mimics the dynamically changing conditions in the stomach and small intestine, and thereby is predictive for what happens in humans, as has been shown by a number of validation studies (refs). It consists of four connected compartments (stomach, duodenum, jejunum and ileum). Transit of the meal through the system is accomplished by peristaltic movements, according to the transit time for the specific food item under investigation. In that sense, we have different protocols for e.g., glass of water, yoghurt, milk, continental breakfast, etc. Moreover, it is possible to mimic different ages of the host. Specific protocols are available for babies, adults and elderly. TIM-1 can be used is two different configurations: for water-soluble and for fat-soluble bioactive compounds. If a product contains both, two separate experiments are performed. This is due to the difference in absorption of water-soluble and fat-soluble compounds.

In a method according to the invention, the provided health data from the individual person are compared with recommended health data. These recommended health data can be as described elsewhere herein or based on common knowledge. One or more individual factors, such as age, gender, genetic factors, life-style or the like may be taken into consideration, if desired. One may also take into account a specific type of medical disorder of which the person is suffering or of which medical disorder the risk of development is aimed to be reduced. Further, one may take gut functioning into consideration; in particular, one may take into consideration whether an individual person adequately digests certain plant-based food product ingredients. If this is not the case, the benefit of consuming a product in accordance with the invention may be limited, because the effect of the consumption on the phytochemical concentrations in blood then tends to be small, if present at all. In such case, an alternative source for a phytochemical of interest can be considered or first the gut functioning is to be improved, in particular by improving the microbiome. Improvement of the microbiome can for instance comprise administration of probiotic bacteria to a person in need thereof or administration of dietary fibre known to stimulate the growth of beneficial bacteria. On the basis of the comparison between the actual health data of the person and recommended data one or more deviation-from-target data are obtained; a deviation from target can be expressed quantitatively as the difference between a provided health data value and the recommended value or qualitatively, e.g. by indicating that a provided health data value imposes a high risk, a limited (yet significant) risk, a low risk or a very low risk. An example for carotenoids is given in Example 1 and elsewhere in the description. The deviation from target is used to determine which phytochemicals should be included in the recommended product. On the basis thereof plant-based ingredients are selected that - in combination - provide the required phytochemicals and the quantities in which the plant-based ingredients are to be combined.

Advantageously, the plant-based ingredients are selected on the basis of their contents of phytochemicals of interest. E.g. advantageously, tomatoes are selected having a high lycopene content, when lycopenes are to be included in the recommended product. When preparing the product, it is further generally preferred that the (fresh) plant-based ingredients or at least a number thereof are obtained from suppliers, preferably directly after harvest (within a few days), in the vicinity of the production facility (if possible), such as within a 300 km radius or a 4 hours transportation distance; this to reduce the risk of degradation of phytochemicals. N.B., in particular, (dried) tea leaves, for making a tea ingredient can advantageously be obtained from a more remote source.

The recommended food product is a physically processed product. Typically, the recommended food product comprises at least one whole fruit or whole vegetable ingredient, i.e. an ingredient consisting of essentially the complete (edible part of the) fruit or the complete (edible part of the) vegetable, which fruit or vegetable typically is mashed/blended) In principle, the recommended food product can be made from a single plant-based ingredient, e.g. tomato. A single ingredient may suffice as a recommendation for a person whose health data are deviating from target data in only one or a few aspects, e.g. only the lycopene level is below a recommended value. In such embodiment, the recommended nutritional product can essentially consist of a single whole fruit or vegetable (in case of a liquid product) or essentially consist of a single whole fruit or vegetable on a solid carrier, such as rice pearls or the like (in case of a solid product); in particular tomato, in case lycopene is below a recommended value, is a suitable single plant-based ingredient. Surprisingly good results have also been achieved with a liquid nutritional product essentially consisting of apple or of apple plus carrier in case of a solid product. Apple (as sole ingredient or in combination with one or more other ingredients) in a nutritional product (for use) according to the invention has amongst others been found very useful for a beneficial effect on microvascular functionality. However typically the product is a combination of two or more plant-based ingredients, preferably 2-50, more preferably 3-30, in particular 4-25, more in particular 5-20 plant-based ingredients. Typically the ingredients are subjected to a size reduction, which can be accomplished during blending of the ingredients. It is also possible to first mash or purée ingredients and thereafter blend the ingredients. The recommended product can have any form. It can be a spoonable product, such as a gel or paste; a drinkable product (a liquid), such as a smoothie; or a solid, such as a bar, beads, pellets, pearls etc. In particular a solid product may comprise a plant-based carrier material. The plant-based carrier material is typically an expanded food material, such as a puffed or extruded cereal, for instance rice, maize or wheat. The carrier material is a material on and/or into which the liquid plant-based nutritional product is loaded (adsorbed). The carrier material typically is open-porous, allowing the liquid plant-based nutritional product to penetrate into the carrier material, in particular when using vacuum coating. Particularly good results have been achieved with (expanded) rice as a carrier material. Rice is typically gluten-free (unlike wheat), which makes the solid product acceptable for persons experiencing a low tolerance for gluten. Moreover, it has been found that a carrier material made from rice shows a high mechanical stability when loaded with the liquid fruit/vegetable product. Various other materials showed a high degree of deterioration (collapse of porous structure) during vacuum coating due to the pressure difference or because of undesired wetting of the material. Furthermore, it is has been found that rice is an inert carrier and can be loaded to a high extent with the liquid plant-based nutritional product. I.e. unlike some carrier materials, e.g. oat, it does not give rise to a noticeable oxidation of phytochemicals. Also from a microbiological perspective, it shows good resistance against microbial growth, which contributes to a good shelf life.

The recommended food product usually comprises ingredients from at least two different edible plant species. At least a major part of the recommended food product is usually formulated from two or more ingredients selected from whole fruits and whole vegetables, in particular 50-100 wt% of the product, preferably 75-100 wt.% of the product, more preferably 90-100 wt.% of the product is composed of whole fruit/vegetable, in particular raw whole fruit/vegetable. In particular for a liquid product according to the invention, advantageously 90-100 wt.% of the total product is composed of whole fruit and whole vegetable ingredients. A product comprising a carrier material is advantageously made from a liquid plant-based product at least substantially consisting of two or more ingredients selected from whole fruit and whole vegetable. It is possible to load the carrier material - in particular rice particles, such as rice pearls - with about 500 g or more of the liquid nutritional product per 100 g carrier material; hereby it is possible to provide a solid product made with more than 50 % whole fruit/vegetable (at least before drying), also when the carrier material as such is not whole fruit/vegetable based.

The use of whole fruit/vegetable has been found to be beneficial to maintain a high content of desired phytochemicals, in comparison to juice only or peeled fruit/vegetable only or fruits/vegetables from which seeds have been removed. Thus, a whole fruit/vegetable based product in accordance with the invention typically has an improved health effect compared to solely using juice from the same fruits/vegetables. There are at least two possible reasons: Parts that are not included in juice may contain high levels of useful phytochemicals (e.g. the skins of blueberry, the skins or seed of blue grapes); further, the presence of parts that are not included in juice may contribute to a more gradual release profile into the blood stream.

Further ingredients, if present, are usually selected from plant-based extracts, which may be liquid or solid, e.g. tea extracts, preferably green tea extracts; parts of fruit or vegetable, e.g. seeds, flowers, peels; and - for solid products - carrier materials, such as cereals.

Solid ingredients may be ground (in particular if dry), or processed in a similar way as or together with whole fruit/vegetable ingredients. If desired, juice or water, preferably water, maybe used as a minor ingredient, e.g. to obtain a food product with a lower viscosity than a product that essentially consists of blended whole fruit/vegetable ingredients. This may e.g. be desired when the provided ingredients have a relatively high solids content, e.g. as can be the case when harvested shortly after a hot and dry period.

If an extract is used, in particular (green) tea extract, it is usually added as the liquid aqueous extract (such as tea for drinking). This facilitates dosing and homogeneous distribution throughout the product. Further, the aqueous extract may provide the water for viscosity adjustment, if desired.

One or more vegetables used as in ingredient in a plant-based product in accordance with the invention are typically vegetable species other than starchy tubers, such as potatoes, starchy roots, such as tapioca, and cereals. Good results have been achieved with products that are essentially free of starchy tubers, starchy roots and cereals, in particular with liquid products that are free thereof. However a cereal, in particular rice, can advantageously be present in a solid plant-based product of the invention, as a carrier material for a blend of other fruit/vegetable ingredients.

The fruit/vegetable ingredients for the plant-based food product in accordance with the invention are generally (ground or mashed/puréed/blended) whole fruits respectively whole vegetables, in particular raw whole fruits respectively whole vegetables. This is considered beneficial for a positive effect of phytochemical. In contrast, using isolated phytochemicals can have a reduced efficacy or even an adverse effect in some instances. If desired, a minor number (typically less than half, in particular only one or two at most) of the ingredients are not whole vegetable/fruit/plant, if considered less practical. E.g. tea is usually added as drinkable tea (an extract from tea leaves). Of a fruit with a large and hard stone (pit), such as cherry, the fruit may be de-pitted before being used as ingredient.

The plant-based food product in accordance with the invention usually comprises at least one, preferably at least two ingredients, in particular at least three ingredients, more in particular at least three, at least four, at least five, at least six, at least seven or more than seven ingredients selected from apple (typically a variety providing isoflavonoids), bell pepper (which may be red, green, yellow or orange), blueberry, bramble, broccoli, brussels sprouts, carrot, cauliflower, cherry (e.g. sour cherry, which is advantageous with respect to gout), chicory, citrus fruits (e.g. orange, tangerine, grape fruit, lemon), endive, *Fabaceae* (e.g. pea, chickpea, beans), grape (in particular blue grape), kale, onion, pear, raspberry, strawberry, spinach, sweet pepper, tomato. The total number of said ingredients is usually 25 or less, in particular 20 or less, more in particular 15 or less. In particular, as illustrated in the Examples, a strong positive effect on blood vessels and a reduction in DNA damage has been shown for a product in accordance with the invention comprising cauliflower, broccoli and brussels sprouts. Such product may essentially consist of these vegetables. In a further advantageous embodiment, the product contains cauliflower, broccoli, brussels sprouts and one or more further ingredients selected from further vegetables, fruits and tea extract.

Further, the presence of pineapple in a blend in accordance with the invention has been found advantageous. Pineapple is a source of bioactive components, such as vitamin C and manganese. Further, it has been found to have a positive effect on consumer appreciation or consumption compliance, in particular in a blend comprising a vegetable, such as a cabbage or brussels sprouts.

Further, the presence of banana in a blend in accordance with the invention has been found advantageous. It is a source of vitamins, such as vitamin B6 and vitamin C and further of minerals, such as potassium and manganese. Further, it has been found suitable to contribute to a more creamy mouthfeel in a liquid product in accordance with the invention , in particular in a liquid product having a noticeable fibrous mouthfeel

When using two or more ingredients, usually at least one fruit ingredient and at least one vegetable ingredient is selected for the formulation of the nutritional product. Besides a possibility to increase diversity in phytochemicals, the inclusion of one or more fruit ingredient with one or more vegetable ingredient has been found to have a positive effect on consumer compliance by persons using the food product.

Two or more of the whole fruit ingredients, whole vegetable ingredients and (green) tea in combination can have an additive health effect or synergistic health effect, for instance a synergistic effect on antioxidant capacity. For example, it has been found that antioxidant capacity of green vegetables (e.g. broccoli) is improved in a mixture further comprising one or more other fruits or vegetables.

Further, said ingredients show satisfactory compatibility; in particular they can be blended in suitable relative amounts without giving rise to unacceptable levels of undesired reactions with one another whereby phytochemical contents are reduced in the mixture, having opposing effects in improving resistance against development of a medical disorder or having other unacceptable effects, such unacceptable olfactory effects. In particular when using whole fruit/vegetable ingredients and optionally (green) tea, active components from one ingredient may have an adverse effect on another whole fruit/vegetable ingredient. Undesirable side-reactions have for instance been noticed when including a substantial amount of cabbage, such as kale, or onion in the plant-based product. Cabbage, bean or onion enzymes may cause a reaction whereby gas is formed. This may not only be undesired from an olfactory viewpoint, but can also lead to a reduction of the phytochemicals content, e.g. because they are a substrate for the enzyme or - in case of a volatile phytochemical - because the gas formation increases vaporisation of the volatile phytochemical. Thus, if unacceptable gas formation is noticed in a product containing cabbage or onion, one may replace at least a part of the cabbage or onion by another vegetable ingredient less prone to form gas. In an advantageous embodiment, the plant-based nutritional product is essentially free of kale, more preferably essentially free of cabbage, and/or essentially free of onion.

When only two ingredients selected from whole fruit ingredients and whole vegetable ingredients (whole fruit/vegetable ingredients) are present in the product, they are usually each individually present in a concentration in the range of 1-99 wt.%, preferably 10-90 wt. %, more preferably 20-80 wt.%, with the proviso that the total of said two ingredients is usually 50-100 wt.%, preferably 75-100 wt.%, more preferably 90-100 wt% of the total nutritional product. When three to six whole fruit/vegetable ingredients are present in the product, they are each individually usually present in a concentration in the range of 1-95 wt.%, preferably 2-90 wt.%, more preferably 3-80 wt.%, with the proviso that the total of said three to six whole fruit/vegetable ingredients is usually 50-100 wt.%, preferably 75-100 wt.%, more preferably 90-100 wt.%. When more than six whole fruit/vegetable ingredients are present in the product, they are usually each individually present in a concentration of less than 95 wt.%, preferably in the range of 1-90 wt.%, more preferably in the range of 3-80 wt.%, with the proviso that the total of said whole fruit/vegetable ingredients is usually 50-100 wt.%, preferably 75-100 wt.%, more preferably 90-100 wt.% . Herein all wt. % are based on the total weight of the product, at least for a product in accordance with the invention without a carrier material, such as a liquid product. For a product on a carrier these values at least apply to the fruit/vegetable ingredients other than the carrier material.

Particular good results have been achieved with a nutritional product comprising at least two ingredients, in particular at least three ingredients, more in particular at least three, at least four, at least five, at least six, at least seven or more than seven ingredients, which are usually mashed or blended, which are preferably raw, selected from the group consisting of whole apple, whole blueberry, whole blue grape, whole bramble, whole brussels sprouts, whole broccoli, whole carrot, whole cauliflower, whole pear, whole raspberry, whole strawberry, whole spinach, whole sweet pepper, whole tomato and green tea (aqueous extract). The total number of said ingredients is usually 15 or less. In particular with such products, which can be grown in a temperate climate (outdoors or in greenhouses) it has been possible to provide a diverse number of phytochemicals, including vitamin C, carotenoids and (iso)flavonoids, without needing to include any citrus fruits, *Fabaceae,* cabbage or onion, whilst being beneficial in accordance with the invention to a person's health. In this embodiment, when essentially free of *Fabaceae,* cabbage or onion, potential drawbacks thereof can be avoided (such as breakdown of bioactive agents/gas formation). Regarding citrus fruits, these are generally grown in (sub)tropical climates, which can logistically expand the distances from which ingredients need to be transported.

In an advantageous embodiment, the recommended nutritional product comprises a green tea ingredient, preferably green tea (aqueous extract), which is in particular a useful antioxidant source, providing protection against cell damage by free radicals. In particular, it contains substantial concentrations of polyphenols and flavonoids, including catechin and isoflavonoids. In a nutritional product (for use) according to the invention its presence is in particular advantageous for an improvement in microvascular functionality.

The invention relates further to a nutritional product that is, comprises, is recommended, or is made (in particular in a process of the invention) with a blend of tomato, carrot, bell pepper and at least one fruit, in particular two to six fruits, preferably selected from the group of apple, blueberry, raspberry, brambles, grape and pear. Such product is in particular suitable for a use according the invention. A blend comprising or consisting of these ingredients is not only beneficial, in particular synergistic, with respect to achieving a health benefit in accordance with the invention, but also has been found to show a particularly good compatibility of ingredients (avoiding undesired levels of side-reactions, e.g. gas formation). The blend may be a drinkable product or it may be immobilised on a carrier, in particular rice pearls. In a preferred embodiment, said blend comprises at 10-30 wt.% tomato, 10-30 wt.% carrot, 10-30 wt.% bell pepper and the balance being said one or more of said fruits. Such blend is also generally appreciated by consumers for its taste; further it has been found to provide high consumer compliance.

Further, the invention relates further to a nutritional product that is, comprises, is recommended, or is made (in particular in a process of the invention) with a blend of blueberry, blue grape, raspberry, bramble, tomato, carrot, green tea (aqueous extract as tea for drinking), bell pepper, tomato, broccoli, cauliflower and brussels sprout. Such product is in particular suitable for a use according the invention. Such blend, which also shows good compatibility in liquid form and in solid product form, is in particular effective for use in increasing resistance against developing a disease of affluence (such as diabetes type 2, a cardiovascular disease) or a cancer (in particular colon cancer, breast cancer or prostate cancer, more in particular colon cancer), reducing the risk of developing such disease or for alleviating a symptom of such disease in a human diagnosed with said disease. It is also particularly beneficial in the improvement of the microbiome. In a preferred embodiment, said blend comprises 4-20 wt.% of each of said ingredients, with the proviso that the total of said ingredients is more than 50 wt.%. In this embodiment, the total of said ingredients is preferably 90-100 wt. %. In a much preferred embodiment, said blend comprises 6-15 wt.% or 6-12 wt.% of each of said ingredients. In this embodiment, the total of said ingredients generally is 75-100 wt. %, preferably 90-100 wt.%. The content of the tea is the content of the liquid tea extract.

To a single person it is possible to recommend a single plant-based nutritional product in accordance with the invention or at least two plant-based nutritional products in accordance with the invention, in parallel or subsequently.

The selection of the actual ingredients and the formulation of the recommended product generally comprises use of information regarding the presence of phytochemicals in available fruits and vegetables and optionally other plant-based ingredients, the input of health data from the person for which the food product is to be recommended and target data (typically data reflecting a healthy state of the body).

The formulation of the plant-based nutritional product, which typically comprises providing a recipe or instruction for preparing the product, optionally followed by the actual production of the product is typically carried out making use of a computer. The computer usually contains a program adapted to perform one or more steps in carrying out the method according to the invention. It usually contains a database with recommended health data. Further it is usually adapted to receive health data from the individual for which the product is to be recommended. Advantageously, the computer contains an algorithm to compare the provide health data of an individual with the recommended health data. Advantageously, it contains a database of food ingredients with typical phytochemical contents. Advantageously, the computer is adapted to select the one or more functional nutritional ingredients for the recommended food product (from the database) and to formulate the recommended product.

In principle it is possible to carry out a method for determining a recommended functional nutritional product according to the invention only once for a single person. The recommendation could then be followed long-term. However, advantageously, health data of a person consuming the recommended nutritional product are provided at intervals and the determination is repeated making use of recent data. Thus, it is possible to adjust the recommendation, dependent on changes in the health data, in time. It is not necessary that each time all types of health data that are provided are the same. Also the intervals between provision of different types of health data can be different. E.g. the provision of retinal eye date and/or microbiome data can be less frequent than the provision of health data that can be obtained from a blood sample or other bodily fluid or tissue sample of the person, such as phytochemical content in blood, or DNA damage or a gene expression profile in blood or cheek saliva. Accordingly, in a preferred method of the invention the steps of a) providing health data of the person, b) comparing the health data provided with the recommended health data profile, c) selecting the one or more nutritional products and d) formulating the recommended food product are carried out repeatedly at intervals - preferably at intervals in the range of 14-366 days, in particular in the range of 28-183 days - and wherein the person is a consumer of the recommended food product.

Advantageously, the recommended nutritional product (formulated in a method for determining the recommended functional nutritional product) is prepared using a process for preparing a plant-based functional nutritional product according to the invention.

The process of preparation of a nutritional product according to the invention is usually carried out whilst the temperature of the material that is being processed ((fresh) fruit/vegetable ingredients, intermediate products such as the blended ingredients, finished product) is kept below 40 °C, preferably in the range of 1-35 ⁰C, more preferably in the range of 5-30 ⁰C, in particular at a temperature up to about 25 ⁰C. A low processing temperature is desired to reduce the risk of unacceptable degradation of phytochemicals. At a higher temperature thermo-instable phytochemicals may decompose, but also reactions between components in the ingredients or oxidation with oxygen is generally accelerated at a higher temperature. N.B., when tea is used as an ingredient, the tea can be made from tea leaves in a conventional way, e.g. by extraction in hot water (typically about 75-100 degrees ⁰C) e.g. for up to 15 min, in particular up to 5 min, after which the tea can be cooled, e.g. to ambient temperature, and then blended with other ingredients. As the skilled person will understand there can be other useful plant-based aqueous extract that can be obtained by extraction in hot water.

Preferably, the period of time between harvest of the fruit/vegetable ingredients and producing the nutritional product from the ingredient(s) is kept short. As the skilled person will understand, the vulnerability of fruits and vegetables to decomposition of phytochemicals and the fruit or vegetable varies significantly between different species or even different varieties. Generally, under suitable storage conditions, it is acceptable to transport the (fresh) fruit or vegetable within 1-3 days from harvest to the production facility where it is further processed with 1-7 days to prepare the product; in particular for dried plant materials (used to make an extract from, such as dried tealeaves) longer storage periods may be desired from a practical view point.

Incoming ingredients are usually subjected to a quality control check. This can be a check by visual means. Pieces of fruit/vegetable showing signs of fungi or bacteria are removed. It is also possible to take samples to determine a phytochemical content. Knowing the content thereof allows fine tuning of the product formulation. The samples can be taken before or after mashing the ingredient. Typically, care is taken that the ingredients are not exposed to excessive temperatures (such as a temperature exceeding 40 ⁰C, with the exception of extracts such as tea, if used) before being further processed. If storage is needed, temperature during storage is usually kept below ambient temperature, preferably in the range of 1-6 ⁰C, in particular in the range of 2-6 ⁰C. Further, the fruit/vegetable ingredients (if they passed the quality screening) are washed before further processing.

The selection of the nutritional product ingredient or ingredients selected from the group consisting of vegetables, fruits, fruit parts, vegetable parts and extracts of plant materials (such as tea) in a process for preparing the plant-based nutritional product is generally based on considerations as described elsewhere herein for the selection of ingredients in the method of determining the recommended nutritional product.

The one or more selected product ingredients are typically mashed. When two or more plant-based ingredients are used, typically a blend of said ingredients is made. The ingredients can be mashed before being blended with another. However, it is also possible to first combine the un-mashed ingredients and then blend them. Blending/mashing is generally done in equipment adapted to mash/blend under relatively low shear to keep temperature low (typically below 40 degrees C). The mashing/blending chamber typically has blending/mashing internals (such as blending/mashing knifes) with a flexible edge, whereby effective blending/mashing can be achieved faster without unacceptable temperature raises. The blending/mashing internals are placed such that 'whipping' of air into the ingredients that are being blended/mashed is low. Processing time is usually kept short, which reduces exposure to air. Hereby it is possible to minimise temperature increase and undesired reactions of the ingredients that are being blended/mashed.

To improve shelf-life stability, the mashed product ingredients or the mashed blend is subjected to an anti-microbial treatment. Pascalisation has been found highly effective as an anti-microbial treatment, whilst phytochemical content is at least substantially maintained. Typically pascalisation is carried out to the mashed ingredient(s), in particular a blend, in a liquid form, usually whilst in a packaging. The packaging typically is a compressible packaging, i.e. it is compressible when exposed to pascalisation pressure. Such packaging is generally known in the art and includes amongst others polyethylene, polypropylene, PET packaging and other packaging made from a thermoplastic material. The packaged nutritional product is placed in a container in which the packaged nutritional product is pressurised. The pressurisation medium can be water or another medium. Usually, the pascalisation is carried out at a pressure in the range of 300 - 1000 MPa, preferably in the range of 450 - 750 MPa, in particular at about 600 MPa (6000 bar). The duration is usually at least 1 min, preferably at least 2 min, in particular about 3 min or more. A pascalisation duration of 10 min or less generally suffices, although it may continue longer if desired. With the pascalisation a satisfactory reduction of undesired colony forming micro-organisms is feasible, whilst maintaining the temperature below 35 ⁰C, preferably below 25 ⁰C. Particularly good results have been achieved with pascalisation in an environment having a temperature in the range of 1-10 ⁰C, more in particular a temperature of about 6 ⁰C. After the anti-microbial treatment has been completed (and packaging), the packaged product is ready for shipment/sale/use etc. A liquid product may be frozen if desired, usually to a temperature in the range of -4 to -30 ⁰C, preferably to a temperature in the range of -15 to -26 ⁰C. Usually, freezing is done after a waiting period, typically 12-36 hours after microbiological treatment, in particular when pascalised.

The mashing/blended generally results in a liquid (drinkable product), although in a specific embodiment a paste or gel (spoonable product) may be produced. The mashed product respectively the blend may be a ready-to-use nutritional product without further processing steps. If desired, the content of one or more phytochemicals of interest in the final product or the blend before antimicrobial treatment (pascalisation) is determined. This is not generally necessary when the product has been made using a process according to the invention, in particular not when the raw ingredients are selected from ingredients of which it has been established that they are rich in phytochemicals of interest or when phytochemical contents have been determined in the raw ingredients; after all, it has been established that the process allows production whilst maintaining at least a substantial part of the phytochemicals, also of thermally instable phytochemicals and phytochemicals prone to undergo side-reactions, such as oxidation in the presence of oxygen.

It is also possible to prepare a solid product from the mash or the blend. In particular preferred is a particulate product, such as a plurality of beads or pearls. Such product is advantageously made using a carrier material, such as a puffed cereal, in particular puffed or extruded rice. A particular suitable process to produce the beads or pearls is an extrusion process, wherein a mash of the carrier material is passed through an extruder and ejected into a reduced pressure chamber. Hereby expanded particles (beads/pearls) are formed. The size in this process can be chosen in a wide range, in particular having a longest diameter from about 1 mm to about 10 mm, more in particular about 2 to about 6 mm. It is also possible though to use carrier materials with a different size.

The carrier material is loaded with the liquid fruit/vegetable mash/blend. Particularly good results have been achieved with vacuum coating, wherein the mash or blend is sucked into the carrier material at sub-atmospheric pressure. This is feasible at a low temperature (below 35 ⁰C), resulting in a high loading rate, typically of between 200 and 800 g liquid fruit/vegetable blend per 100 g carrier material, in particular when using rice pearls or equivalent carrier material, preferably of 400-600 g liquid fruit/vegetable blend per 100 g carrier material

In a specific embodiment, the invention thus relates to a particulate solid nutritional product comprising a carrier material, such as extruded rice, and a dried fruit/vegetable mash. Such product is typically obtainable by a process according to the invention, in particular a process wherein the carrier material is provided with the dried mash by vacuum coating.

.A high loading rate of the carrier material is desirable for high bioactivity per weight of product. In terms of dry weight of the final carrier-based solid nutritional product, the solid nutritional product generally contains at least about 20 g ingredients (dried fruit/vegetable/tea ingredients) other than carrier material per 100 g carrier material, preferably at least about 30 g ingredients (dried fruit/vegetable/tea) other than carrier material per 100 g carrier material, more preferably at least about 33 g ingredients (dried fruit/vegetable/tea) other than carrier material per 100 g carrier material. In practice, the dry carrier-based nutritional product generally contains up to about 80 g ingredients (dried fruit/vegetable/tea) other than carrier material per 100 g carrier material, in particular up to about 50 g ingredients (dried fruit/vegetable/tea) other than carrier material per 100 g carrier material, more in particular about up to about 45 g or up to about 40 g ingredients (dried fruit/vegetable/tea) other than carrier material per 100 g carrier material.

The prepared nutritional product may be packaged in a unit-dose or in a package for multiple use (e.g. providing enough for a total daily multiple dosage of the plant-based product for a single person). Conveniently, a package for an individual person may comprise 40-1000 g of product, preferably about 100-1000 g of product, e.g. about 100 g, about 250 g, about 330 g, about 400 g, about 500 g or about 1000 g.

However, it is also possible to package the nutritional product in a larger amount per package, such as a package of up to about 10 kg product. E.g. the contents of a bag or other container of about 10 l can still be pascalised satisfactorily. Such volume packaging can in particular be interesting for producing a blend of a very high number of ingredients, in particular at least 10, at least 11 or at least 12. The diversity of ingredients provides a diversity of phytochemicals and can be offered to a plurality of people (e.g. in a restaurant, bar or canteen). Such offering can advantageously be done as an initial step prior to starting a personalised recommendation on the basis of personal health data.

As mentioned above, the invention further relates to the plant-based nutritional product as described herein, for use in increasing resistance against developing a medical disorder in a human, reducing the risk of developing a medical disorder in a human or for alleviating a symptom of a medical disorder in a human diagnosed with said medical disorder, wherein the plant-based nutritional product to be administered is formulated using a method according to the invention. Said human is thus typically the individual person to which the nutritional product is recommended.

An indication for the effectivity of a product for use in accordance with the invention in a single person can e.g. be provided by determining health data after a period e.g. of two weeks, a month, three months, six months or a year of consuming the product regularly (preferably daily, preferably in two times over the day) and comparing those with the health data just before starting the first consumption. E.g. increased phytochemical contents in blood, reduction of DNA damage in white blood cells or cheek saliva, improved vascularisation of the retina, or an increase of bacteria in the small intestine are supportive of the effectivity. As illustrated in the examples, effects can already be observes after several weeks. Further evidence can be provided by cohort studies wherein a group of persons consuming the product regularly (preferably daily) and a control group of persons are followed for, e.g., 1 year or more, preferably for at least 3 years or at least 5 years and comparing the incidence of a medical disorder or a group of medical disorders. The skilled person will be able to design a study with a suitable group of people, duration etc based on common general knowledge, the references cited herein and the contents of the present disclosure.

Good results are achieved with the plant-based nutritional product, for said use wherein the medical disorder selected from the group consisting of metabolic syndrome, insulin resistance and diabetes mellitus type 2.

Further, good results are achieved with the plant-based nutritional product, for said use wherein the medial disorder is a cardiovascular disease.

Further, good results are achieved with the plant-based nutritional product, for said use wherein the medial disorder is a cancer, preferably a cancer selected from colon cancer, breast cancer and prostate cancer, in particular a colon cancer. In particular, the cancer is a non-genetic form of cancer.

In a further advantageous embodiment, the plant-based nutritional product is for a medical use, wherein the medical disorder is COPD. The inventors realised that a positive effect on blood vessels that is achievable with a product in accordance with the invention is also advantageous in relation to treating (e.g. alleviating a symptom) of COPD or reducing the risk of developing COPD, in particular in view of a positive effect of a nutritional product has been found to have on blood vessels. E.g. a positive effect on micro-vessels diameter in the lungs (like pulmonary arterioles and capillaries) can contribute to a better blood passage and uptake of oxygen into the bloodstream. Accordingly, the inventors in particular consider that in particular the teaching herein relating to cardiovascular diseases or effects on (retinal) blood vessels may also be applied when aiming to treat or reduce the risk of developing COPD.

It is envisaged that the plant-based nutritional product is suitable for use in in increasing resistance against developing one or more other medical disorders, reducing the risk of developing of one or more other medical disorders or for alleviating a symptom of one or more other medical disorder in a human diagnosed with said medical disorder; for instance an eye disorder, a gastro-intestinal disorder, osteoporosis, gout (for which a product comprising (sour) cherry is considered useful) or pathological obesity. Examples of symptoms that may be alleviated can for instance be selected from: high blood pressure, blood vessel dilation problems, reduced insulin sensitivity, inflammation.

The nutritional product used or for use in accordance with the invention is generally administered (consumed) orally. The nutritional product is usually (to be) administered at least about once a week, preferably- on average - at least four times per week, more preferably - on average - at least seven times per week. In particular good results with respect to a medical use, more in particular a medical use selected from increasing resistance against a medical disorder, reducing the risk of developing a medical disorder or for alleviating a symptom of a medical disorder are achieved when a nutritional product is administered daily in accordance with the invention. A regular and frequent administration contributes to maintaining the phytochemicals at an advantageous concentration in blood throughout the week, preferably throughout the day. Accordingly, it is particularly preferred to administer the plant-based nutritional product in accordance with the invention - on average - at least twice a day, in particular two, three or four times per day. The total daily dosage is advantageously a supplementation of at least 25 %, in particular of at least about 50 % to the daily recommended intake of fruits and vegetables by nutritionists.

A minimum dosage per administration is usually at least about 100 g, preferably at least about 125 g, in particular at least about 150 g. A single dosage is usually up to about 400 g, preferably up to about 300 g, more in particular about 250 g or less. A recommended average daily dosage is usually in the range of in the range of about 200-500 g, preferably in the range about 250-500 gram of the plant-based nutritional product, e.g. the liquid plant-based nutritional product in 3, 4 or 5 doses of about 100 ml; the liquid plant-based nutritional product in 2, 3 or 4 or 5 doses of about 125 ml the liquid plant-based nutritional product in 2 doses per day of either about 200 ml or about 250 ml; about 400 g plant-based nutritional product in a single dose per day, two doses per day, thee doses per day or four doses per day; or about 250 g plant-based nutritional product in either in a single dose per day, two doses per day, thee doses per day or four doses per day.

As used herein, the singular forms "a", "an" and "the" are intended to include the plural forms as well, unless it clearly follows from the context that only the singular form is intended. The term " or" includes any and all combinations of one or more of the associated listed items, unless the context clearly indicates otherwise (e.g. if an "either ....or" construction is used). It will be understood that the terms "comprises" and "comprising" specify the presence of stated features but do not preclude the presence or addition of one or more other features. It will be further understood that when a particular step of a method is referred to as subsequent to another step, it can directly follow said other step or one or more intermediate steps may be carried out before carrying out the particular step, unless specified otherwise or clear from the context that no intermediate steps can be present. Likewise, it will be understood that when a connection between structures or components is described, this connection may be established directly or through intermediate structures or components unless specified otherwise.

In the context of this application, the term "about" includes in particular a deviation of 20 % or less from the given value, more in particular 10%, more in particular 5% or less.

The term "(at least) substantial(ly)" or " (at least) essential(ly)" is generally used herein to indicate that it has the general character or function of that which is specified. When referring to a quantifiable feature, this term is in particular used to indicate that it is at least 75 %, more in particular 90 % or more, even more in particular 95 % or more of the maximum of that feature. The term 'essentially free' is generally used herein to indicate that a substance is not present (below the detection limit achievable with analytical technology as available on the effective filing date) or present in such a low amount that it does not significantly affect the property of the product that is essentially free of said substance.

When referring to a "liquid product" this means a product that is fluid at a temperature of 25 ⁰C. A liquid product may be frozen e.g. for storage or in a specific embodiment be consumed in a frozen state.

Weight percentages (wt.%) are generally weight to weight percentage based on total weight, unless it is specified otherwise or it follows otherwise from the context.

For the purpose of clarity and a concise description, features are described herein as part of the same or separate embodiments, however, it will be appreciated that the scope of the invention may include embodiments having combinations of all or some of the features described.

The invention will now be illustrated by the following examples.

### EXAMPLE 1 RISK PROFILING BASED ON CAROTENOID VALUES

The total carotenoid content based risk factors of developing one or more diseases of affluence, such as a cancer, metabolic syndrome/insulin resistance/diabetes mellites type 2 or a cardiovascular disease are as follows:
- less than 1.5 µM : a high risk,
- 1.5-2.5 µM: limited yet significant risk,
- 2.5-4.0 µM: low risk
- more than 4.0 µM very low risk.

The levels of the four carotenoid marker groups and the total level of carotenoid of a blood sample of an individual were determined. Results are shown in the following table.

| *Carotenoid group* | Concentration (µM) | Concentration in 95 % of population | Conclusion |
|---|---|---|---|
| α-β-carotene | 0.37 | (0.1-0.7 µM) | low yellow/orange vegetable value |
| Lycopene | 0.58 | (0.2-0.8 µM) | average red vegetable value |
| β-Cryptoxanthin | 0.078 | (0.06-0.4 µM) | low fruit consumption |
| Lutein/Zeaxanthin | 0.06 | (0.1-0.59 µM) | Very low green vegetable consumption |

A blend of blueberry, blue grape, raspberry, bramble (or blue berry), tomato, carrot, green tea (liquid aqueous extract, as drinking tea), bell pepper (sweet red pepper), tomato, broccoli, cauliflower and brussels sprout was recommended, each ingredient present in a content of about 6 to about 12 wt.%.

### EXAMPLE 2 METHOD COMPRISING A RETINAL SCAN

Of humans, images of the fundus of the right eye were taken before and after a two-week intervention with a smoothie which consisted of a particular blend of 400 grams of whole fruits and whole vegetables per day (either as a drink or blend immobilised on rice pearls). Although a positive effect was noticeable for each blend, the positive effect was most pronounced in humans treated with a blend as recommended in Example 1 (rich in polyphenols, anthocyanins, carotenoids and glucosinolates).

Retinal analysis was performed on the images using Iflexis software and mean arteriole width (CRAE) and mean venule width (CRVE) of the 6 largest vessels were calculated. In addition, the AVR (CRAE/CRVE) which is a ratio that considers both dimensions, was also determined. Following linear mixed model statistical analysis and 108 comparisons pre and post-intervention, a significant difference was seen in all measures. CRAE increased by 3.6%, CRVE decreased by 1.3%, and AVR increased by 4.8%. These changes suggest a protective effect, as arterial narrowing is seen in hypertension, coronary artery disease in women, and is associated with diabetes risk, while larger venules are seen in obesity, hypertension, and diabetic retinopathy, and lower AVR is associated with diabetes incidence and heart disease mortality.

### EXAMPLE 3: PREFERRED EXAMPLES OF NUTRITIONAL PRODUCTS

The following liquid blends and solid product were formulated.

| | |
|---|---|
| **1** | Apples, green tea |
| **2** | Blue berries, blue grape, blackberries, raspberry |
| **3** | Tomato, carrots, sweet red pepper |
| **4** | Broccoli, cauliflower, Brussels sprouts |
| **5** | Apples, green tea, blue berries, blue grape, blackberries, raspberry |
| **6** | Apples, green tea, blue berries, blue grape, blackberries, raspberry, tomato, carrots, sweet red pepper |
| **7** | Apples, green tea, blue berries, blue grape, blackberries, raspberry, tomato, carrots, sweet red pepper, broccoli, cauliflower, Brussels sprouts |

| ***Pearls*** | |
|---|---|
| **8(ref)** | 100% rice pearl |
| **9** | Rice pearls (8) plus 40 wt.% coating of blend 7 |

The preparation of the liquid blends comprised_selecting the product ingredients from the group consisting of whole vegetables, whole fruit and tea; blending the selected product ingredients, typically in about equal amounts by weight; packaging the obtained blend in a compressible container (plastic bottle); pascalisation of the packaged blend. The temperature was kept below 35 ⁰C throughout the process.

The solid product was prepared by loading expanded particles made of rice (rice pearls) with a liquid blend obtained in a process as described in the previous paragraph, using vacuum coating, whilst maintaining the temperature below 35 ⁰C

### EXAMPLE 4: HUMAN DIETARY INTERVENTION STUDY

A human dietary intervention study was designed, wherein different liquid blends of whole fruit(s) & whole vegetable(s), as described in Example 3, with typically about equal amounts of each ingredient) were consumed four times a day at a total daily dosage of 400 ml (4 x 100 ml), including a blend based on the recommendation in Example 1. Further a number of subjects consumed the rice pearls loaded with blend 7 of Example 3 (i.e. product 9 of Example 3).

The study had a randomised controlled cross-over repeated measures design.

The test group was formed of 200 healthy, consenting, volunteers, familiar with the confidential nature of the study. They had a BMI in the range of 18.5-27 kg/m² (mean BMI +/- SD: 22.8 +/- 2.1 at the start) and an age in the range of 18-60 years (mean age +/- SD: 28 +/- 11). Exclusion criteria included: alcohol abuse up to 6 months prior to study; currently smoking or smoking till 3 months or less prior to study; current presence of symptoms to a disease of the gastro-intestinal tract, kidney, liver, heart or lungs, or disease related to the endocrine system; HIV infection, hepatitis; anaemia; pregnant women; use of dietary supplements or antibiotics or other 3 months or less prior to study; medication other than contraceptives; food allergy relevant to dietary intervention; vegetarians and vegans; highly physically active people (> 8 hrs. per week heavy exercise/sports); participants of other intervention studies during this intervention period.

The monitored health data included
- ex-vivo induced hydrogen peroxide lymphocyte damage
- gene expression profiling using a CometAssay^{®}
- antioxidant capacity of blood plasma
- retinal vascular health data (using a retinal camera, cf. Example 2)
- height and weight

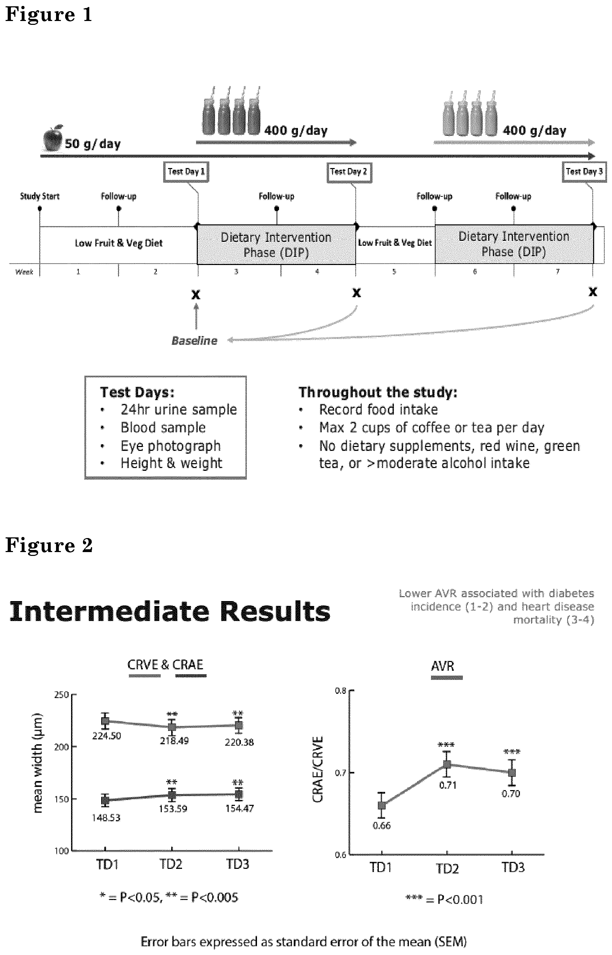

The time-line of the first seven weeks is shown in Figure 1. After the initial period, the administration can be continued by alternating between two weeks of supplementing the diet of the test persons and one week not supplementing. Or the test persons' diet can be supplemented throughout the remainder of the test.

Throughout the study, the participants recorded food intake, had maximally two cups of either coffee or black tea per day; they did not take none of dietary supplements, red wine and green tea. Their alcohol intake, if any, was not more than moderate.

### Intermediate results

Average changes in body weight compared to average body weight at test day 1 (66.5 kg +/- 12.4 kg) were not statistically significant at test day 2 (2 weeks later ) or at test day 3 (seven weeks later): 66.7 kg +/- 12.4 kg at test day 2 and 65.7 kg +/- 12.3 kg at test day 3.

However, retinal health data already showed statistically significant improvement at the second test day. See Figure 2. The increased AVR (CRAE/CRVE) is supportive on increasing the resistance respectively reducing the risk of developing diabetes or a cardiovascular disorder, because a low AVR is associated with diabetes incidence and heart disease mortality. In Figure 2 (left hand diagram) CRVE is the top line; CRAE the bottom line.

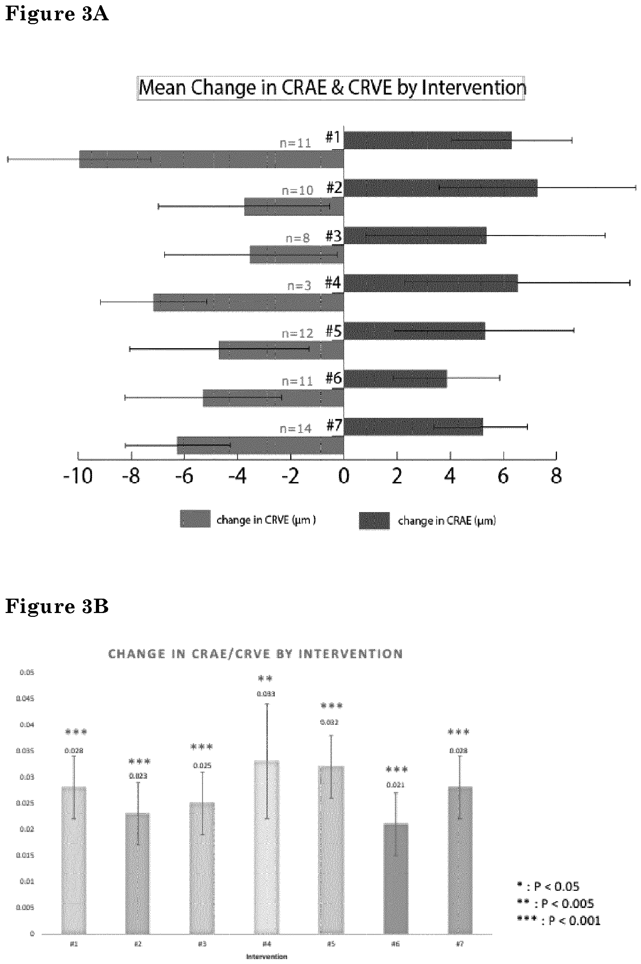

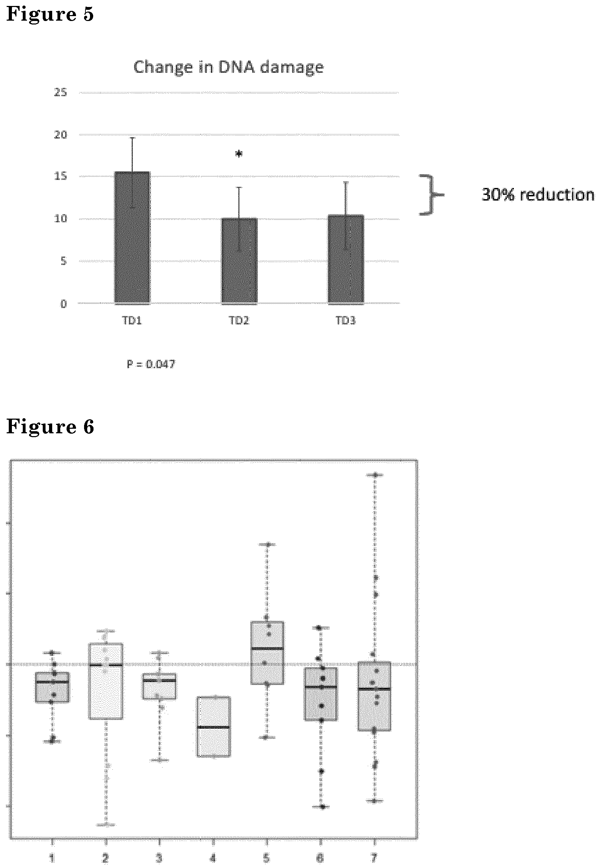

Intervention numbers in the Figures 3A, 3B and 6 correspond to the product numbers in Example 3.

Figure 3A shows mean changes in CRAE and CRVE by the different interventions.

Figure 3B shows changes in the ratio CRAE to CRVE by the various interventions. It is shown that each of the products had a positive effect. Strongest reduction were obtained with product 4 (a blend of broccoli, cauliflower and brussels sprouts, i.e. vegetables only) and product 5.

Further, Figures 5 and 6 illustrate the positive effect of consumption of a plant-based nutritional product in accordance with the invention on reducing DNA damage. Figure 5 shows that - on average - tail DNA damage was reduced by 30 % after two weeks (TD2) and seven weeks (TD3). Figure 6 shows changes vs. baseline (TD1). For each of the seven tested products, there were subjects in which DNA damage was reduced. Strongest reduction - on average - was obtained with product 4 (a blend of broccoli, cauliflower and brussels sprouts, i.e. vegetables only). E.g. product 7, containing the same vegetables as product 4, product 1 and product 6 were also effective. The average effect on DNA damage was less than for product 4, but any of these products can - dependent on the intended user - be a recommended alternative for product 7, e.g. if a vegetable-only product is less attractive to consume for that user. Thereby recommending, e.g., product 7, can contribute to consumption compliance.

The results (not shown) with solid product 9 (rice pearls loaded with product 7) were in line with the results obtained with the liquid product 7.

## Claims

1. A method for determining a recommended functional nutritional product for an individual person based on health data of that person, wherein
a) one or more health data of the person are provided, said health data comprising
• a content of at least one phytochemical, preferably a plurality of phytochemicals, in a blood sample of the person;
b) said health data are compared with recommended health data;
c) one or more plant-based nutritional product ingredients are selected for the recommended food product on the basis of the recommended health data, said ingredients containing phytochemicals; and
d) the functional nutritional product is formulated from said one or more plant-based nutritional product ingredients, which recommended functional nutritional product is plant-based.

2. Method according to claim 1, wherein the health data further comprise at least one, at least two, at least three or each of the following health data:
- retinal blood vessel condition data of the person;
- a gene-expression profile of the person;
- data regarding the sensitivity to DNA damage upon exposure to oxidative stress of blood cells of the person; and
- gastro-intestinal health data of the person, preferably microbiome data of the person.

3. Method according to any of the preceding claims, wherein the health data comprise the contents in the blood sample of one or more phytochemicals, preferably at least two phytochemicals, more preferably at least four phytochemicals selected from the group of polyphenols, flavonoids, isoflavonoids, anthocyanins, diallyl sulphide, phytoestrogens, terpenoids, carotenoids, limonoids, phytosterols, glucosinolates, mono-unsaturated fatty acids, polyunsaturated fatty acids, caffeine, carnitine, choline, co-enzyme Q10, creatine, dithiolthiones, prebiotics, taurine, vitamins, preferably one or more selected from carotenoids, polyphenols, anthocyanes, glucosinulates, co-enzyme Q10, vitamin A, vitamin D and vitamin E.

4. Method according to any of the claims 1-3, wherein at least the phytochemical contents in the blood sample of the following carotenoids are determined: (1) the carotenes content (which may be the total of alpha-carotene and beta-carotene or the individual contents of alpha-carotene and beta-carotene); (2) the lycopenes content; (3) beta-cryptoxanthin content; and (4) the total content of lutein plus zeaxanthin or the individual contents of lutein and zeaxanthin; and wherein the said contents are separately compared with recommended contents, or wherein said contents are used to determine the total carotenoid content in the blood and the total carotenoids content in the blood is compared with a recommended total carotenoids content,
wherein in said method preferably further the contents of vitamin A, D and E , the content of co-enzyme Q10 and the ratio ubiquinone to ubiquinol is determined and compared with their recommended content.

5. Method according to any of claims 1-4, wherein two or more nutritional product ingredients are selected from whole fruits and whole vegetables, in particular raw whole fruits and raw whole vegetables, which may be mashed or blended, which whole fruits and whole vegetables are selected from the group consisting of apple, blueberry, blue grape, bramble, brussels sprouts, broccoli, carrot, cauliflower, onion, pear, raspberry, strawberry, spinach, bell pepper and tomato.

6. Method according to claim 5, wherein the nutritional product is selected from the group consisting of
- liquid blends at least substantially consisting of tomato, carrot, bell pepper and at least one fruit;
- nutritional products composed of a dried blend and a carrier material, which dried blend at least substantially consists of tomato, carrot, bell pepper and at least one fruit, which carrier material preferably is made of rice;
- liquid blends at least substantially consisting of blueberry, blue grape, raspberry, bramble, tomato, carrot, green tea, bell pepper, broccoli, cauliflower and brussels sprout; and
- solid nutritional products composed of a dried blend and a carrier material, wherein the dried blend at least substantially consists of blueberry, blue grape, raspberry, bramble, tomato, carrot green tea extract, bell pepper, tomato, broccoli, cauliflower and brussels sprout, which carrier material preferably is made of rice.

7. Method according to any of the claims 1-6, wherein said blend comprises 6-12 wt.% whole blueberry, 6-12 wt.% whole blue grape, 6-12 wt.% whole raspberry, 6-12 wt.% whole bramble, 6-12 wt.% whole tomato, 6-12 wt.% whole carrot, 6-12 wt.% green tea, 6-12 wt.% whole bell pepper, 6-12 wt.% w whole broccoli, 6-12 wt.% whole cauliflower and 6-12 wt.% whole brussels sprout.

8. Method according to any of the claims 1-7, wherein a) the health data of the person are fed into a computing device, and wherein b) the comparing of provided health data with the recommended health data profile, c) the selection of the one or more nutritional ingredients, and d) the formulation of the recommended nutritional product comprises the use of the computing device.

9. Method according to any of the claims 1-8, wherein the steps of a) providing health data of the person, b) comparing the health data, c) selecting the one or more nutritional ingredients and d) formulating the recommended food product are carried out repeatedly - preferably at intervals in the range of 14-366 days, in particular in the range of 28-183 days - and wherein the person, at least after the first time of carrying out said steps, is a consumer of the recommended food product.

10. Process for preparing a plant-based functional nutritional product, which plant-based nutritional product is as defined in any of the preceding claims, which plant-based nutritional product is optionally formulated using a method according to any of the preceding claims, wherein the process comprises
- selecting one or more, preferably two or more, more preferably three or more nutritional product ingredients selected from the group consisting of whole vegetables, whole fruit, tea and parts thereof;
- mashing the selected product ingredient or ingredients thereby obtaining a mash, preferably blending in case of two or more ingredients thereby obtaining a blend;
- preferably packaging the obtained mash respectively the obtained blend;
- subjecting the mash respectively blend to pascalisation whilst in a compressible packaging or other container;
- thereby obtaining the plant-based nutritional product;
which process is carried out at a temperature of about 35 ⁰C or less, preferably a temperature in the range of 1-30 ⁰C.

11. Process according to claim 10, wherein an open porous particulate carrier material, preferably made from rice, is loaded with the mash respectively blend, preferably the pascalised mash respectively blend, by vacuum coating, wherein the mash respectively blend is dried and a dry plant-based nutritional product is obtained, in which process preferably the weight to weight ratio the mash respectively blend to carrier material is at least about 400 g per 100 g carrier material, more preferably about 500 to about 750 g per 100 g carrier material.

12. Plant-based nutritional product selected from the group consisting of
- dry particulate plant-based nutritional product, obtainable by a process according to claim 11, wherein preferably the carrier material is a plurality of rice pearls;
- solid plant-based nutritional products composed of a dried blend and a carrier material, preferably obtainable by a method according to claim 11, wherein the blend at least substantially consists of green tea and the following whole fruit/vegetable ingredients: blueberry, blue grape, raspberry, bramble, carrot, bell pepper, tomato, broccoli, cauliflower and brussels sprout, which carrier material preferably is made of rice;
- liquid plant-based nutritional products, obtainable by a method according to claim 10; and
- liquid plant-based nutritional products at least substantially consisting of a blend of blueberry, blue grape, raspberry, bramble, tomato, carrot, green tea, bell pepper, broccoli, cauliflower and brussels sprout, preferably obtainable by a method according to claim 10.

13. Plant-based nutritional product according to claim 12, wherein said blend is a blend of 6-12 wt.% whole blueberry, 6-12 wt.% whole blue grape, 6-12 wt.% whole raspberry, 6-12 wt.% whole bramble, 6-12 wt.% whole tomato, 6-12 wt.% whole carrot, 6-12 wt.% green tea, 6-12 wt.% whole bell pepper, 6-12 wt.% whole broccoli, 6-12 wt.% whole cauliflower and 6-12 wt.% whole brussels sprouts.

14. A plant-based nutritional product, for use in increasing resistance against developing a medical disorder in a human, reducing the risk of developing a medical disorder in a human or for alleviating a symptom of a medical disorder in a human diagnosed with said medical disorder, wherein the plant-based nutritional product is selected from the group consisting of
- plant-based nutritional products formulated using a method according to any of the claims 1-9;
- plant-based nutritional products obtainable by a method according to claim 10 or 11; and
- plant-based nutritional products according to claim 12 or 13.

15. A plant-based nutritional product for a use according to claim 14, wherein the medical disorder is selected from the group consisting of
- retina disorders;
- - cancers, preferably a cancer selected from the group consisting of colon cancer, prostate cancer and breast cancer, in particular colon cancer;
- metabolic syndrome, insulin resistance and diabetes mellitus type 2;
- cardiovascular diseases; and
- COPD.
